# EUROPEAN PATENT SPECIFICATION

(11) **EP 2 438 031 B1**
(45) Date of publication and mention of the grant of the patent: **12.04.2017**
(21) Application number: 09840183.9
(22) Date of filing: 02.06.2009
(51) Int. Cl.: A01K 67/033, B32B 5/02, B32B 27/18, B32B 3/26, B32B 27/12, B32B 27/32

(54) **A TETRA VERMI BED AND A PROCESS FOR COMPOSTING AGRICULTURAL WASTE**
TETRA-WURMBETT UND VERFAHREN ZUR KOMPOSTIERUNG VON LANDWIRTSCHAFTLICHEM ABFALL
LIT DE VERS DE TERRE À STRUCTURE "TÉTRA" ET PROCÉDÉ DE COMPOSTAGE DE DÉCHETS AGRICOLES

(43) Date of publication of application: 11.04.2012
(73) Proprietor: Dhoot, Kishorilal Ramnath, Maharashtra (IN); Dhoot, Kamlesh Kishrorilal, Maharashtra (IN)
(72) Inventor: Dhoot, Kishorilal Ramnath, Maharashtra (IN); Dhoot, Kamlesh Kishrorilal, Maharashtra (IN)
(74) Representative: Virdee-Crofts, Kulwinder Kaur
(86) International application number: PCT/IN2009/000312
(87) International publication number: WO 2010/140155

(56) References cited:
- EP-A1- 0 567 210
- WO-A1-92/04303
- WO-A1-2006/084611
- FR-A1- 2 913 014

## Description

### Field of the Invention:

This invention relates to the field of composting waste into manure and more particularly to the field of composting agricultural waste into nutrient-rich manure in an earthworm-friendly, portable, foldable tetra vermi bed, supported on fourteen grounded poles through fourteen totally heat sealed pockets.

### Background of the Invention:

Following works are cited to pinpoint inherent drawbacks encountered in composting the agricultural waste.

US Patent 702952 - This work calls for mechanical actions and is operationally complex for semi-literate/illiterate farmers. The present invention requires no energy input.

US Patent 6488733 - This work entails composting in pits and is restricted to permanently dug pits. The present invention allows portability to site -where agricultural waste is collected - and ease in installation. The present invention is even suitable for sites such as hilly areas which are not easily accessible or pose barriers.

US Patent - 11331973 and 10403816 - These works are based on use of animal urine for composting. The composting method and the apparatus are both different from the present invention, which places no reliance on animal urine for composting and yields solid manure for easy end-application.

US Patent 10872174 - This work relates to composting sewage in an apparatus, using a plurality of tanks and the required piping. The present invention allows portability to site of agricultural waste and requires no plurality of tanks and no complex handling.

US Patent 6576462 - This work relates to vermi culture composting device for organic/kitchen waste, relying upon sunlight partially to control temperature for composting. Container for composting is painted oppositely in dark and light colours for absorbing or reflecting sunlight. The present invention is entirely different from the cited works in terms of no painting of alternate sides with black shade being required.

FR 2913014 - This work employs a plurality of tanks with perforated bottoms and covered with air holes. It employs dendrodrilus rubidus worm for composting. The present invention employs a portable multilayered fabric tank for composting.

US Patent Des. 332,163 This work employs a vermi composting container, which is fitted with a plurality of perforated pipes near bottom end of the container for introducing water. The present work yields manure, which is easy to handle in end-application and relies upon a composting bed made of a composite fabric.

U. S. No. 7018831 - This prior work relates to a composting apparatus which comprises a plurality of stackable composting drawers and a plurality of receivers, each receiver arranged under one unit of said plurality of drawers. The present invention is different from the cited work.

U. S. No. D332163 - This prior work relates to a composting container, which is rigid and employs laterally a plurality of horizontal, perforated, hollow rods. Said rods ends are covered with perforated screen to vent out gases/liquid.

The present invention is different in terms of foldable bed, which - when inserted in fourteen vertically heat-sealed pockets- support the composite fabric bed above ground level in a rectangular shape.

U. S. No. 6,576,462 - This prior work relates to a composting device, which is made of four rectangular plywood rigid panels. The panels exteriorly are painted in varying colors. The present invention employs a portable, foldable bed, which is supported above the ground by means of inserting onto top end of fourteen grounded wooden poles fourteen fully heat- sealed pockets of the foldable bed. The present invention is very much different from the cited work.

### Objects of the Invention

One object of the present invention is to allow portability of the composting apparatus to the sites, having agricultural waste and the ease in installation of the composting apparatus. It is implied that the apparatus is light in weight,
easy to carry to the waste cites and quick in installation.

Another object of the present invention is to yield high-quality manure that is easy to handle, to pack in conventional bags and to economically transport.

Another object of the present invention is to propagate reproduction of the culture and to protect the culture against pilferage, predators such as ants, snakes and rodents.

Another object of the present invention is to eliminate, in composting, requirement for mechanical energy and complex, plurality of tanks, vessels and piping system.

### Brief Description of the Drawings

Drawing # 1 illustrates a perspective view of tetra vermi bed (100).
Drawing # 2 illustrates main panel (100-1).
Drawing # 3 illustrates the corner eyelet (100-10..100-13)) and flap fold (100-14...100-17).
Drawing # 4 illustrates the installation of vermi wash drain pipe (83) and the dug-out pit (85).
Drawing # 5 illustrates joining by heat-sealing the side panels (100-2, 100-3) with the main panel (100-1) and joining two longitudinally extending portions of the main panel (100-1) with the side panels (100-2, 100-3).
Drawing # 6 illustrates sealing in vertical configuration of the longitudinally extending portions of the main panel (100-1) with the two lateral sides each of the side panels (100-2, 100-3) and sealing HDPE net over the drain (71).
Drawing # 7 illustrates inverted U-shaped pocket (80-13, 80-14) of main panel (100-1) and half-rounded opening at bottom to facilitate insertion of wooden pole (81)
Drawing # 8 illustrates interior view of vertical corner joint (100-4... 100-7).
Drawing # 9 illustrates pocket U-cutting of main panel (100-1) and main panel (100-1) top edge rope folding.
Drawing # 10 illustrates inserting tetra vermi bed (100) onto grouted wooden pole (81-1..81-14).
Drawing # 11 illustrates HDPE net sealing over window cutting (70-1..70-6).
Drawing # 12 illustrates inverted-U shaped pockets (80-1...80-12), windows (70-1...70-6) heat sealed on side panel (100-2, 100- 3).
Drawing # 13 illustrates side panel (100-2, 100-3) vertical sealing (100-4...100-7)
Drawing # 14 illustrates fourteen wooden poles (81-1...81-14) grouted in dug-out holes (82-1...82-14) in ground (95) respectively.

### Detailed description of the invention

Tetra vermi bed (100) is constructed of a seven-layered, chemically-treated, U.V.-stabilized, composite HDPE woven fabric (90), which is prepared by alternately laminating a layer of LDPE (92) in between two layers of HDPE (91). The composite fabric (90) has three layers of HDPE, which are made from HDPE granules, added with UV-stabilizing compound, and which alternate with four LDPE layers (signifying the word 'Tetra' of the product's name). The combined seven layers are laminated by extrusion coating into a single composite fabric (90). An feature of the present invention is the use of the UV-stabilized, composite fabric (90) in construction of tetra vermi bed (100). UV-stabilizing compound is added 3-5% by weight to the granules to prevent cracking of the composite fabric (90), caused due to exposure to sunlight.

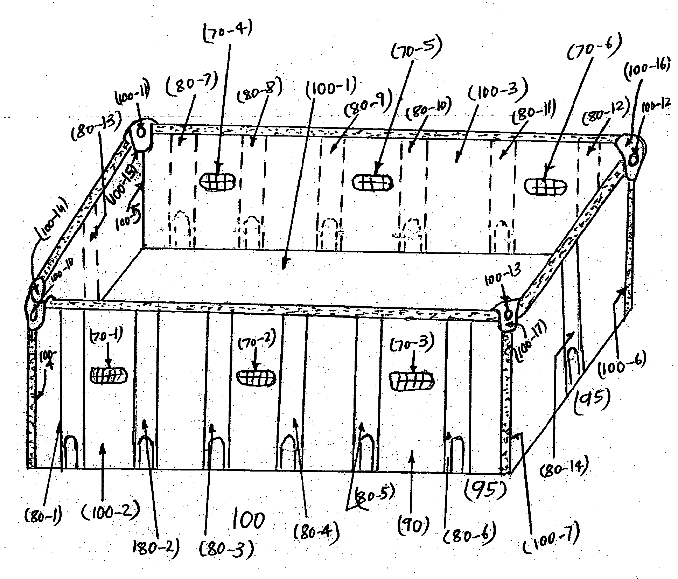

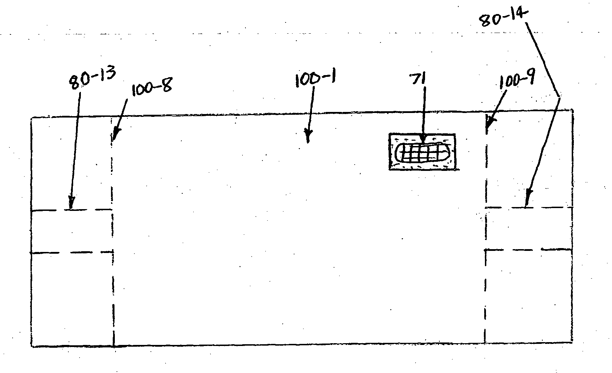

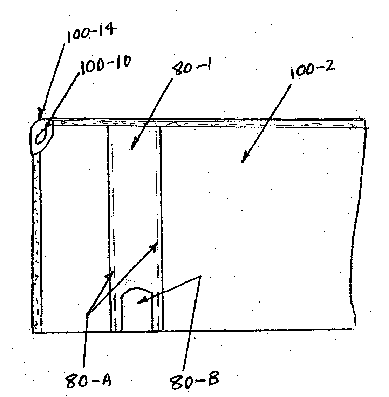

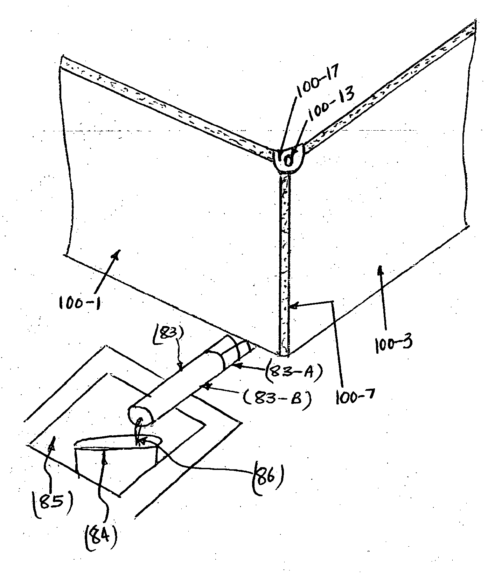

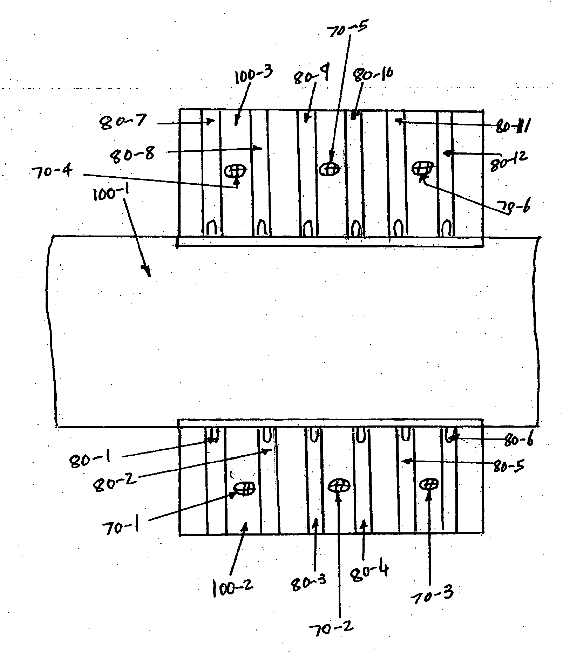

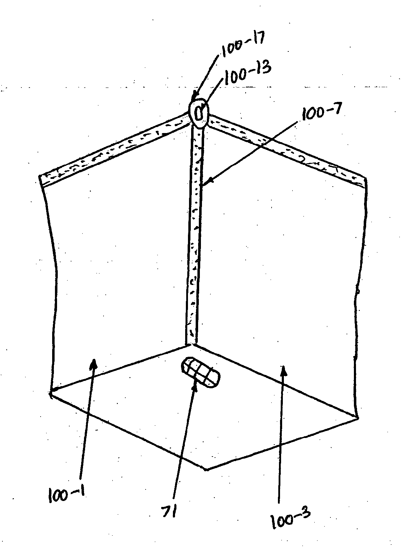

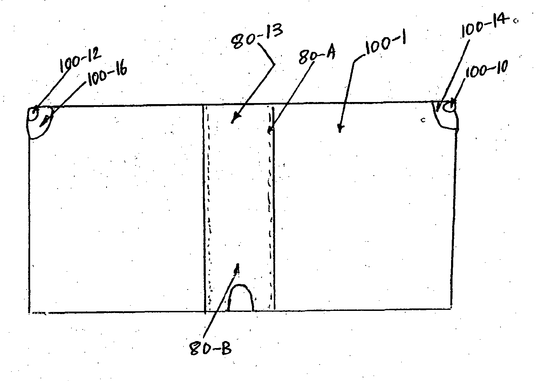

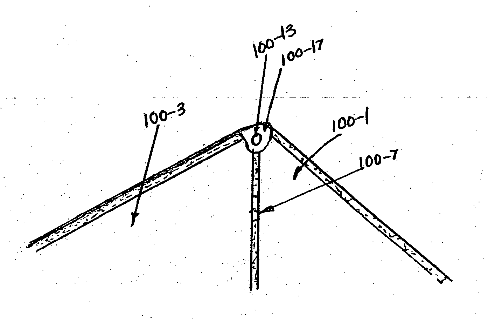

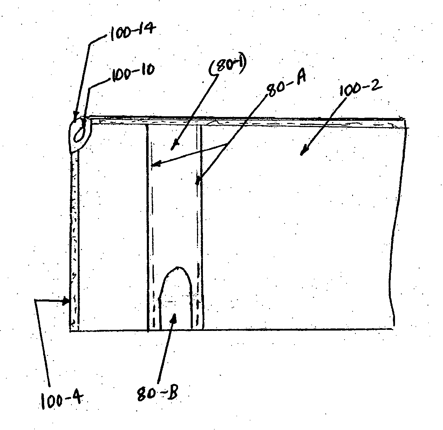

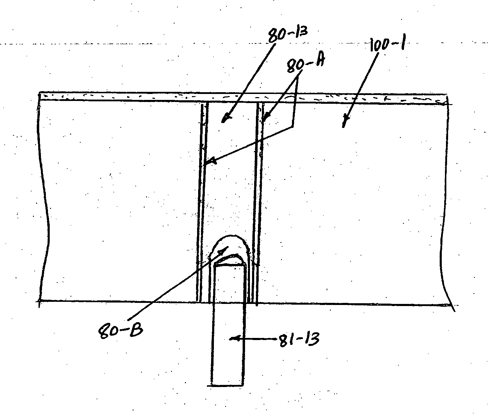

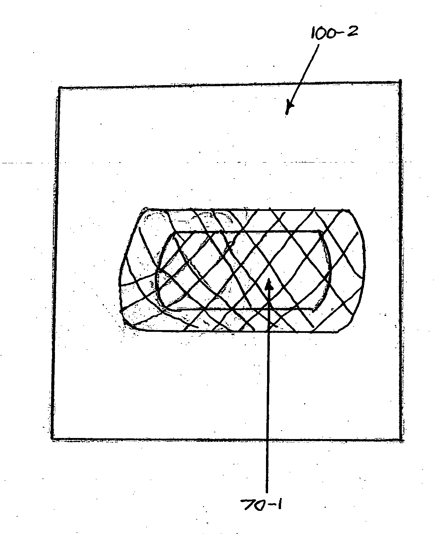

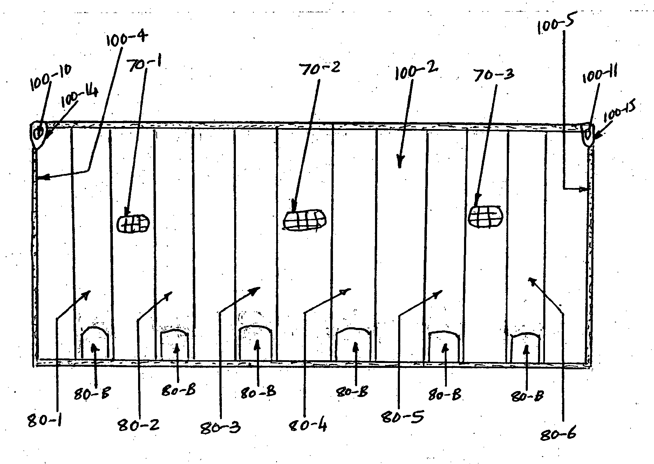

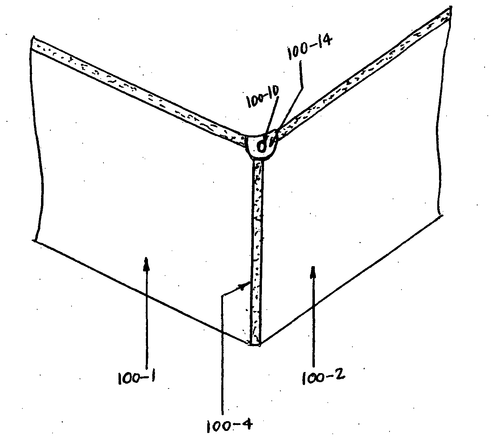

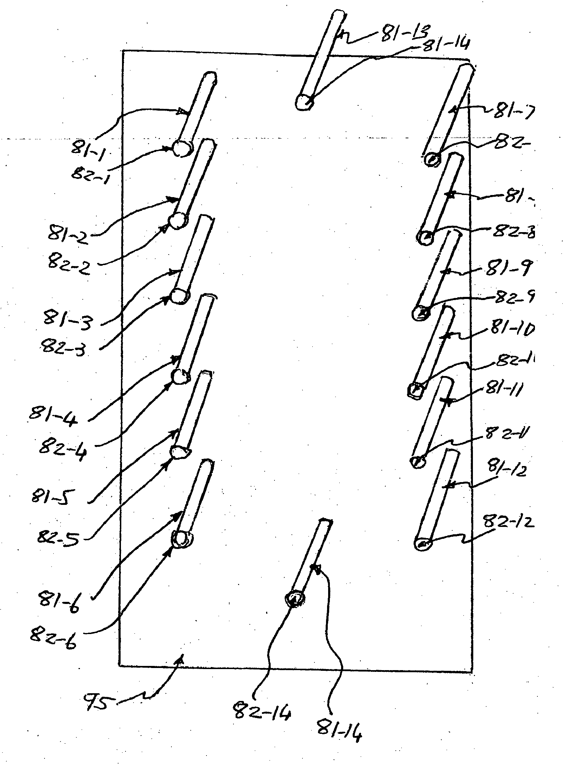

Tetra vermi bed (100) is constructed of a main panel (100-1) of said composite fabric (90), admeasuring 5. 105 meters x 1.27 meters (16 feet 9 inches by 4 feet 2 inches) and of a twin side panel (100-2, 100-3) of said composite fabric (90), admeasuring 3.88 meters x 0.60 meters (12 feet 9 inches by 2 feet). Each side panel (100-2, 100-3) 5 is processed, as explained below, before it is joined with the main panel (100-1):
Tetra vermi bed (100) is firmly erected over laid ground (95) - provided with a gradient of 0.15 meters over 3.65 meters (6 inches over 12 feet) by means of fourteen pockets (80-1..80-14), of which six pockets (80-1..80-6) exteriorly and
perpendicularly to longitudinal axis are heat-sealed on the side panel (100-2) of the tetra vermi bed (100), as illustrated in the accompanying drawing # 1, and the other six pockets (80-7..80-14) exteriorly and perpendicularly to the longitudinal axis are heat-sealed on the other side panel (100-3) of the tetra vermi bed (100).The thirteenth pocket (80-13) exteriorly and parallel to the longitudinal axis is heat-sealed, as illustrated in the accompanying drawing # 1, on one lateral side of the main panel (100-1) of the tetra vermi bed (100) and the fourteenth pocket (80-14) exteriorly and parallel to the longitudinal axis is heat-sealed on the other lateral side of the main pan el (100-1) of the tetra vermi bed (100). The fourteen pockets (80-1 to 80-14) are of inverted U-shape, open at bottom end and closed at the top end such that straight, 0.03 meters (1^{1/2} inch) diameter wooden pole (81) is easily slid through 0.15 meters (6 inch) clearance provided in each of said fourteen pockets (80-1 to 40- 5 14) for the firm installation of the tetra vermi bed (100) over the well-laid ground (95). The second feature of the present invention is the fourteen, inverted u-shaped pockets (80-1 to 80- 14), as explained above.

Each U-shaped pocket (80-1 to 80- 14) is - during process of attachment - heat-sealed simultaneously on two sides thereof (80A) onto the panels (100-1, 100-2, 100-3) of the tetra vermi bed (100), leaving a 0.15 meters (six inch) clearance (80B) along the entire length of the U-shaped pocket being attached (80-1 to 80- 14), as illustrated in the accompanying drawings # 7, 9 and 12. The third feature is characterized by the twin-sided heat-sealing (80a) of fourteen pockets (80-1 to 80-14) and allowing 0.15 meters six inch clearance (80b) along the entire length of the fourteen pockets (80-1 to 80-14). tetra vermi bed (100) breathes by ventilating out hot air generated within the mass being composted and facilitating circulation of fresh air into the composting mass through six (70-1... 70-6) windows. Three windows (70-1... 70-3) are provided on the side panel (100-2) of the tetra vermi bed (100) and the other three windows (70-4... 70-6) are provided on the other side panel (100-3) of tetra vermi bed (100). The windows (70-1... 70-6) are marked on the respective side panels (100-2, 100-3) of the tetra vermi bed (100) and the marked areas with round corners are cut out from the respective side panels. An HDPE net is sealed onto the cut portion, as illustrated in the accompanying drawing # 11.

A drain (71) is provided in the main panel (100-1) of the tetra vermi bed (100), as illustrated in the accompanying drawing # 4, by marking, cutting out the marked portion and sealing with an HDPE net (71). The fourth feature of the present invention is characterized by the provision of six windows (70-1... 70-6) - in the form of HDPE net - on the side panels (100-2, 100-3) and a drain outlet (71) - in the form of HDPE net
- on the main panel (100-1), as explained above.

The side panels (100-2, 100-3) and the main panel (100-1) have their respective longitudinal and the lateral ends - farthest to the U-shaped mouths of the pockets (80-1 to 80-14) - roped end-to-end. Roping the main panel (100-1) and the side panels (100-2, 100-3) characterize the fifth feature of the tetra vermi bed (100), as explained above.

The inverted U-shaped pockets (80-1 to 80-12), after heat sealing with the side panels (100-2, 100-3), have their other end cut into U-shape, folded over the roped edge of the respective panels (100-2, 100-3) for anchoring and heat-sealed to the interior side of the panels (100-2, 100-3). U-pockets (80-13, 80-14) are similarly anchored over the two lateral sides of the main panel (100-1). Anchoring the inverted, u- shaped pockets (80-1..80-14) over the panels (100-1.. 10-3), as explained above, characterize the sixth feature of the tetra vermi bed (100).

The side panels (100-2, 100-3) at the un-roped longitudinal ends are heat-sealed with the main panel (100-1), thereby anchoring the twelve U-pockets (80-1...80-12) at their respective lower ends , as illustrated in the accompanying drawing # 5. The two lateral sides of the side panels (100-2,100-3) are sealed in vertical configuration with the respective extending sides of the main panel (100-1) to form four joints (100-4 to 100-7), that form the four vertical sides of the tetra vermi bed (100). The vertical joints (100-4... 100-7) are each folded sideways and then heat-sealed over the folds to impart extra strength against tear, as illustrated in the accompanying drawing # 6. The seventh feature of the tetra vermi bed is characterized by the joining, by heat-sealing, the two side panels (100-2, 100-3) with the main panel (100-1) and strengthening the joints - that form the vertical sides of the tetra vermi bed (100) - by folding sideways the vertical joints at (100-4 to 100-7) and heat-sealing over the folds the four vertical joints (100-4 to 100-7). The tetra vermi bed (100) is fully constructed in a foldable form and is ready for installation.

The novelty of the present invention lies in the installation of the tetra vermi bed (100) by grouting fourteen straight, 1.01 meters (40 inch) long, circular
wooden poles (81-1 to 81-14) to 0.45 meters (18 inches) depth from the ground level (95)- as illustrated in the accompanying drawing # 14 - sliding downward the fourteen pockets (80-1 .... 80-14) over the grouted fourteen wooden poles (81-1...81-14) to the points closest to the ground surface such that the poles (81-1...81-14) firmly hold the four vertical sides of the tetra vermi bed (100), with the central portion of the main panel (100-1) forming the bottom of the tetra vermi bed (100) between the two fold lines (100-8, 100-9), as illustrated in the accompanying drawing # 2.

Four eyelets (100-10 to 100-13) are provided at top four corner points of the tetra vermi bed (100) to facilitate roping and the eyelets are covered with flaps (100-14 to 100-17), as illustrated in the accompanying drawing # 3, to protect the roping from damage by the edges of the eyelets (100-10 to 100-13).

A drain (71) is fabricated at the marked portion of the main panel (100-1) by cutting out the marked portion with round corners and heat-sealing an HDPE net over the cut portion. A HDPE drain pipe (83), semi-circularly cut-open along a part length, is fitted under the drain (71) such that the tetra vermi bed (100) fits neatly over the cut-open length (83-A) of the pipe (72) and the remainder uncut portion (83-B) is buried in the ground and placed above the container (84), as illustrated in the accompanying drawing # 4, which is placed in the dug-out pit (85) to collect vermi wash liquid (86). The drain (71) is positioned at the lowest point of the gradient of the laid ground (95).

The eighth feature of the present invention is characterized in the construction of the drain (71) and by the positioning of the drain pipe (83), semi-circularly cut open along a part length (83-a) for placement under the drain (71) of the tetra vermi bed (100).

One embodiment of the present invention is constructed along the following technical parameters of specific values, as summarized below, for the tetra vermi bed (100) of size 3.65 meters x 1.21 meters x 0.60 meters (12 feet x 4 feet x 2 feet) to perform optimally. They are as follows:

### Main Panel:

```
1. inverted U-pockets (80-13, 80-14) 2 number, size - 7.31
       meters x 2.43 meters (24 inches x 8 inches)
 2. drain (71) 0.08 meters x 0.03 meters (3.5 inch x 1.5 inch)
 3. top edge rope folding 0.05 meters (2 inch)
 4. (a) main panel (100-1) to side } 0.05 meters (2 inch)
       panel (100-2, 100-3) sealing }
       width }
  (b) four end vertical joint } 0.05 meters (2 inch)
      sealing, formed between }
        lateral end of side panel }
        (1002, 100-3) and }
        extended portion of main }
        panel (100-1) }
     (c) fold sealing width } 0.05 meters (2 inch)
```

### Side panel:

```
5. inverted U-pockets (80-1...80-12)
        0.60 meters x 0.20 meters (24 inch x 8 inch)
 6. window (70-1...70-6) 0.20 meters x 0.11 meters ( 8 inch x 4.5
         inch)
 7. pocket to window distance 0.11 meters (4.5 inch)
 8. vertical comer-to-pocket 0.12 meters (5 inch)
       distance
 9. pocket U-cut 0.08 meters x 0.11 meters (3.5 inch x
         4.5 inch)
 10. top edge rope fold 0.05 meters (2 inch)
 11. colour exterior - green, interior - white
 12. tensile strength of the } warp - 190 kgf
       composite fabric (90) } weft - 125 kgf
 13. tensile strength at bottom 100 kgf
       Seal
 14. tensile strength at pocket seal 100 kgf
 15. tear strength of fabric warp - 200 kgf
                                      weft - 140 kgf
 16. bursting strength of fabric 35kg/sq.cm.
 17. water penetration should pass
 (specifications 11 to 17 apply to main panel (100-1), side
 panels (100-2, 100-3) and pockets (80-1...80-14) )
 18. accelerated aging test } should pass
 270 at 70 deg. C, 7 days } should pass
 19. chemical resistance test should pass
 20. environment stress and }
       resistance to cracking }
       - at 50 degree C, 24 hours should pass
       - at 90 degree C, 48 hours should pass
```

## Claims

1. a stitch less, heat-sealed, HDPE substrate- tetra LDPE -layered, vermi composting bed (100), constructed of a composite fabric (90), said composite fabric (90) made of UV-stabilized, four, hot extrusion coated, LDPE layers, said four layers laminated and alternating with UV-stabilized, three, HDPE substrate (woven fabric) layers, said tetra vermi bed (100) comprising:
(i) a main panel (100-1), of said composite fabric (90), admeasuring 5.105 meters x 0.60meters (16 feet, 9 inches x 2 feet);
(ii) two side panels (100-2, 100-3), each said side panel of said composite fabric (90), admeasuring 3.88 meters x 0.60 meters (12 feet 9 inches x 2 feet) such that
(iii)
(a) the size of said tetra vermi bed (100), made of said seven-layered composite fabric (90) admeasures 12 feet long 4 feet wide x 2 feet high;
(b) said vermi bed (100) has an internal volumetric capacity to contain 2.714 cubic meters (96 cubic feet) volume of waste; and
(c) the color of said tetra vermi bed (100) exteriorly is green and interiorly is snow white;
(iv) fourteen, inverted U-shaped pockets (80-1 .... 80-14) made of said seven-layered composite fabric (90), each said pocket open with inverted U-shaped mouth at bottom end thereof and closed at top end thereof by anchoring by roped end of said side panels (100-2, 100-3) or of said main panel (100-1);
(v) fourteen round, straight, 0.99 meters (39 inch) long wooden poles (81-1... 81-14), each said pole grouted at a pre-marked holes (82-1...82-14) to 0.45 meters (18 inches) depth below the laid ground surface (95) and projecting above the ground surface (95) to a height of 0.5 meters ( 21 inches);
(vi) six windows (70-1 .... 70-6), made by cutting out respective pre-marked portion from said side panels (100-2, 100-3) and heat sealing HDPE net, over said cutout portions with round corners, each said window admeasuring 0.20 meters x 0.11 meters (8 inches x 4.5 inches);
(vii) a drain (71), cut open in marked portion of said main panel (100 -1) and covered with a HDPE net over the drain (71) opening, said drain (71) resting atop a semi-circularly, cut-open pipe (83) along part length (83-A) and buried for the remainder length (83-B) in soil under said drain (71) of said tetra vermi bed (100), said drain (71) admeasuring 0.088 meters x 0.03 meters (3.5 inches x 1.5 inches).

2. A stitch less, heat-sealed, HDPE substrate- tetra LDPE-layered, vermi composting bed (100) as claimed in claim 1, wherein said six pockets (80-1..80-6)
(a) admeasure 0.60 meters x 0.20 meters (24 inches x 8 inches); and
(b) are heat-sealed on said green exterior side of said side panel (100-2) in a position, perpendicular to longitudinal axis of said side panel (100-2) of said tetra vermi bed (100).

3. A stitch less, heat-sealed, HDPE substrate -LDPE -layered, vermi composting bed (100) as claimed in claim 1, wherein the other said six pockets 80-7..80-12)
(a) admeasure 0.60 meters x 0.20 meters (24 inches x 8 inches); and
(b) are heat-sealed on said green exterior side of said side panel (100-3) in a position, perpendicular to longitudinal axis of said side panel (100-3) of said tetra vermi bed (100).

4. A stitch less, heat-sealed, HDPE substrate- tetra LDPE -layered, Vermin composting bed (100) as claimed in claim 1, wherein said thirteenth pocket (80-13)
(a) admeasures 0. 60 meters x 0.20 meters (24 inches x 8 inches); and
(b) is heat-sealed on said green exterior surface and near one lateral side of said main panel (100-1), in position parallel to the longitudinal axis of said main panel (100-1).

5. A stitch less, heat-sealed, _HDPE substrate- tetra LDPE -layered, vermi composting bed (100) as claimed in claim 1, wherein said fourteenth pocket (80-14)
(a) admeasures 0.60 meters x 0.20 meters (24 inches x 8 inches); and
(b) is heat-sealed on said green exterior surface and near said other lateral side of said main panel (100-1), in position parallel to the longitudinal axis of said main panel (100-1).

6. A stitch less, heat-sealed, substrate_HDPE- tetra LDPE -layered, vermi composting bed (100) as claimed in claim 1, wherein said windows (70-1......70-3) are centrally cut open from said side panel (100-2) in 0.20 meters x 0.11 meters ( 8 inch x 4.5 inch) openings with round corners which are heat-sealed with HDPE nets.

7. A stitch less, heat-sealed, HDPE substrate- tetra LDPE -layered, vermi composting bed (100) as claimed in claim 1, wherein said windows (70- 4...70-6) are centrally cut open from said side panel (100-3) in 0.20 meters x 0.11 meters (8 inch x 4.5 inch) openings with round corners which are heat-sealed with HDPE nets.

8. A stitch less, heat-sealed, HDPE substrate- tetra LDPE -layered, vermi composting bed (100) as claimed in claims 1 and 2 to 5, wherein said pockets (80-1...80-14) are heat-sealed on two sides (80-A) of said pockets (80-1...80-14) over a 0.15 meters (6 inch) clearance (80-B) along the entire length of said pockets (80-1...80-14).

9. A stitch less, heat-sealed, HDPE substrate- tetra LDPE -layered, vermi composting bed (100) as claimed in claim 1, wherein
(a) said side panels (100-2, 100-3) are rope-folded by 0.05 meters (2 inches) at one longitudinal end, that is farthest from said inverted, U-shaped mouth of said pockets (80-1...80-12); and
(b) said main panel (100-1) is rope-folded by 0.05 meters (2 inches) at both lateral ends, which are farthest to said inverted, U-shaped mouth of said pockets (80-13, 80-14).

10. A stitch less, heat-sealed, HDPE substrate- tetra LDPE -layered, vermi composting bed (100) as claimed in claim 1, wherein
(i) said each side panel (100-2, 100-3) at un-roped longitudinal side is heat sealed with one longitudinal side of said main panel (100-1)
(ii) two lateral sides of each side panel (100-2, 100-3) are each heat-sealed with extended longitudinal sides of said main panel (100-1) to form vertical corner joints (100-4... 100-7) that are folded sideways by 0.05 meters (2 inches) and sealed over folds for additional strength.

11. A stitch less, heat-sealed, HDPE substrate-tetra LDPE -layered, vermi composting bed (100) as claimed in claim 1, wherein
(i) said window (70-1, 70-2, 70-3, 70-4, 70-5, 70-6) each is 0.11 meters (4.5 inch) away from said adjoining, inverted U-shaped pocket [(80-1, 80-2), (80-3,80-4), (80-5, 80-6), (80-7,80-8), (80-9, 80-10), (80-11,80-12)] respectively and
(ii) said inverted U-shaped pocket (80-1, 80-6, 80-7, 80-12) each is 0.12 meters (5 inch) away from said vertical corner joint (100-4, 100-5, 100-6, 100-7) respectively

12. A stitch less, heat-sealed, HDPE substrate- tetra LDPE -layered, vermi composting bed as claimed in claims 1, 2 and 3, wherein
(a) each said inverted U-shaped pocket (80-1...80-12) at U-shaped one end is heat-sealed - along with said side panel (100-2, 100-3) - to said main panel (100-1) and at said closed other end is anchored over the roped top side of said side panels (100-2, 100-3) by cutting out a U-shaped portion off the closed other end and heat sealing it with said side panel (100-2, 100-3);
(b) each said inverted U-shaped pocket (80-13, 80-14) at closed other end is anchored over the roped top side of said main panel (100-1) by cutting out a U-shaped portion off the closed other end and heat sealing it with said main panel (100-1).

13. A stitch less, heat-sealed, HDPE substrate- tetra LDPE -layered, vermi composting bed (100), as claimed in claim 1, is installed on
well laid ground (95) having a gradient of 0.15 meters (6 inches) over 3.65 meters (12 feet) by
(i) marking correctly fourteen locations for grouting said fourteen wooden poles (81-1...81-14) on an area selected for installation, said selected area of said well-laid ground (95) admeasuring 4.57 meters x 1.82 meters (15feet x 6 feet);
(ii) digging said marked positions into fourteen holes (82-1...82-14) each said hole admeasuring 0.07 meters (3 inches) in diameter x 0.45 meters (18 inches) deep;
(iii) grouting each said wooden pole (81-1...81-14) in each said dug hole (82-1...82-14) such that each said pole (81-1.. 81-14) rise above said well-laid ground (95) to a height of 0.53 meters (21 inches);
(iv) inserting said pockets (80-1...80-14) onto said respective wooden poles (81-1...81-14) of said tetra vermi bed (100) simultaneously and sliding down gradually said tetra vermi-bed (100) over said wood wooden poles (81-1...81-14) till said tetra vermi bed (100) touches said well laid ground (95) all along its periphery.
(v) fitting under said drain (71) said drain pipe (83) such that said tetra vermi bed (100) sits atop said semi-circularly cut-open portion (83-A) of said drain pipe (83) and placing the other end (83-B) of said drain pipe (83) above a container (84), placed in a dug-out pit (85) for collecting vermi wash liquid (86).

14. A stitch less, heat-sealed, _HDPE substrate- tetra LDPE -layered, vermi composting bed (100), as claimed in claim 1, wherein four eyelets (100-10 to 100-13) are provided at top four corner points of said tetra vermi bed (100) for roping the top edges of said side panels (100-2, 100-3) and of said main panel (100-1), said eyelets (100-10...100-13) being covered with folding flaps (100-14 to 100-17) to protect the roping from damage by the edges of said eyelets (100-10 to 100-13).

## Patentansprüche

1. Nahtloses, verschweißtes, HDPE-Substrat-Tetra-LDPE-lagiges Wurmkompostierungsbett (100), konstruiert aus einem Verbundstoff (90), wobei der genannte Verbundstoff (90) aus UV-stabilisierten vier heißextrusionsbeschichteten LDPE-Lagen gefertigt ist, wobei die genannten vier Lagen laminiert und abwechselnd mit UV-stabilisierten drei HDPE-Substrat-(Gewebe)-Lagen laminiert sind, wobei das genannte Tetra-Wurmbett (100) Folgendes umfasst:
(i) ein Hauptpaneel (100-1) aus dem genannten Verbundstoff (90) mit den Maßen 5,105 Meter x 0,60 Meter (16 Fuß, 9 Zoll x 2 Fuß);
(ii) zwei Seitenpaneele (100-2, 100-3), wobei jedes genannte Seitenpaneel des genannten Verbundstoffs (90) die Maße 3,88 Meter x 0,60 Meter (12 Fuß 9 Zoll x 2 Fuß) hat, so dass
(iii)
(a) die Größe des genannten Tetra-Wurmbetts (100) bestehend aus dem genannten siebenlagigen Verbundstoff (90) die Maße 12 Fuß lang 4 Fuß breit x 2 Fuß hoch hat;
(b) das genannte Wurmbett (100) eine Innenvolumenkapazität zum Aufnehmen eines Abfallvolumens von 2,714 Kubikmetern (96 Kubikfuß) hat; und
(c) die Farbe des genannten Tetra-Wurmbetts (100) außen grün und innen schneeweiß ist;
(iv) vierzehn Taschen (80-1...80-14) in der Form eines umgekehrten U bestehend aus dem genannten siebenlagigen Verbundstoff (90), wobei jede genannte Tasche mit der Mündung in der Form eines umgekehrten U am unteren Ende davon offen und am oberen Ende davon geschlossen ist, durch Verankern mit einem verseilten Ende der genannten Seitenpaneele (100-2, 100-3) oder des genannten Hauptpaneels (100-1);
(v) vierzehn runde, gerade Holzpfähle (81-1...81-14) mit einer Länge von 0,99 Metern (39 Zoll), wobei jeder genannte Pfahl in einem vormarkierten Loch (82-1...82-14) mit einer Tiefe von 0,45 Metern (18 Zoll) unter der ausgelegten Bodenfläche (95) vergossen ist und über der Bodenfläche (95) auf eine Höhe von 0,5 Metern (21 Zoll) vorsteht;
(vi) sechs Fenster (70-1...70-6), gefertigt durch Ausschneiden eines jeweiligen vormarkierten Abschnitts aus den genannten Seitenpaneelen (100-2, 100-3) und Verschweißen des HDPE-Netzes über den genannten ausgeschnittenen Abschnitten mit abgerundeten Ecken, wobei jedes genannte Fenster die Maße 0,20 Meter x 0,11 Meter (8 Zoll x 4,5 Zoll) hat;
(vii) einen Abfluss (71), der in einem markierten Abschnitt des genannten Hauptpaneels (100-1) aufgeschnitten und mit einem HDPE-Netz über der Abflussöffnung (71) bedeckt ist, wobei der genannte Abfluss (71) auf einem halbkreisförmigen, aufgeschnittenen Rohr (83) über eine Teillänge (83-A) ruht und über die restliche Länge (83-B) in der Erde unter dem genannten Abfluss (71) des genannten Tetra-Wurmbetts (100) vergraben ist, wobei der genannte Abfluss (71) die Maße 0,088 Meter x 0,03 Meter (3,5 Zoll x 1,5 Zoll) hat.

2. Nahtloses, verschweißtes, HDPE-Substrat-Tetra-LDPE-lagiges Wurmkompostierungsbett (100) nach Anspruch 1, wobei die genannten sechs Taschen (80-1...80-6)
(a) die Maße 0,60 Meter x 0,20 Meter (24 Zoll x 8 Zoll) haben; und
(b) auf der genannten grünen Außenseite des genannten Seitenpaneels in einer Position lotrecht zur Längsachse des genannten Seitenpaneels (100-2) des genannten Tetra-Wurmbetts (100) verschweißt sind.

3. Nahtloses, verschweißtes, HDPE-Substrat-LDPE-lagiges Wurmkompostierungsbett (100) nach Anspruch 1, wobei die anderen genannten sechs Taschen (80-7...80-12)
(a) die Maße 0,60 Meter x 0,20 Meter (24 Zoll x 8 Zoll) haben; und
(b) auf der genannten grünen Außenseite des genannten Seitenpaneels (100-3) in einer Position lotrecht zur Längsachse des genannten Seitenpaneels (100-3) des genannten Tetra-Wurmbetts (100) verschweißt sind.

4. Nahtloses, verschweißtes, HDPE-Substrat-Tetra-LDPE-lagiges Wurmkompostierungsbett (100) nach Anspruch 1, wobei die genannte dreizehnte Tasche (80-13)
(a) die Maße 0,60 Meter x 0,20 Meter (24 Zoll x 8 Zoll) hat; und
(b) auf der genannten grünen Außenfläche und in der Nähe einer lateralen Seite des genannten Hauptpaneels (100-1) in einer Position lotrecht zur Längsachse des genannten Hauptpaneels (100-1) versiegelt ist.

5. Nahtloses, verschweißtes, HDPE-Substrat-Tetra-LDPE-lagiges Wurmkompostierungsbett (100) nach Anspruch 1, wobei die genannte vierzehnte Tasche (80-14)
(a) die Maße 0,60 Meter x 0,20 Meter (24 Zoll x 8 Zoll) hat; und
(b) auf der genannten grünen Außenfläche und nahe der genannten anderen lateralen Seite des genannten Hauptpaneels (100-1) in einer Position parallel zur Längsachse des genannten Hauptpaneels (100-1) verschweißt ist.

6. Nahtloses, verschweißtes, Substrat-HDPE-Tetra-LDPE-lagiges Wurmkompostierungsbett (100) nach Anspruch 1, wobei die genannten Fenster (70-1...70-3) zentral aus dem genannten Seitenpaneel (100-2) in Öffnungen von 0,20 Meter x 0,11 Meter (8 Zoll x 4,5 Zoll) mit abgerundeten Ecken ausgeschnitten sind, die mit HDPE-Netzen verschweißt sind.

7. Nahtloses, verschweißtes, HDPE-Substrat-Tetra-LDPE-lagiges Wurmkompostierungsbett (100) nach Anspruch 1, wobei die genannten Fenster (70-4...70-6) aus dem genannten Seitenpaneel (100-3) in Öffnungen von 0,20 Meter x 0,11 Meter (8 Zoll x 4,5 Zoll) mit abgerundeten Ecken ausgeschnitten sind, die mit HDPE-Netzen verschweißt sind.

8. Nahtloses, verschweißtes, HDPE-Substrat-Tetra-LDPE-lagiges Wurmkompostierungsbett (100) nach den Ansprüchen 1 und 2 bis 5, wobei die genannten Taschen (80-1...80-14) auf zwei Seiten (80-A) der genannten Taschen (80-1...80-14) über einen Freiraum (80-B) von 0,15 Metern (6 Zoll) über die gesamte Länge der genannten Taschen (80-1...80-14) verschweißt sind.

9. Nahtloses, verschweißtes, HDPE-Substrat-Tetra-LDPE-lagiges Wurmkompostierungsbett (100) nach Anspruch 1, wobei
(a) die genannten Seitenpaneele (100-2, 100-3) um 0,05 Meter (2 Zoll) an einem Längsende seilgefaltet sind, das sich am weitesten von der genannten Mündung der genannten Taschen (80-1...80-12) in der Form eines umgkehrten U entfernt befindet; und
(b) das genannte Hauptpaneel (100-1) um 0,05 Meter (2 Zoll) an beiden lateralen Enden seilgefaltet ist, die sich am weitesten von der genannten Mündung der genannten Taschen (80-13, 80-14) in der Form eines umgekehrten U entfernt befindet.

10. Nahtloses, verschweißtes, HDPE-Substrat-Tetra-LDPE-lagiges Wurmkompostierungsbett (100) nach Anspruch 1, wobei
(i) jedes genannte Seitenpaneel (100-2, 100-3) auf einer seillosen Längsseite mit einer Längsseite des genannten Hauptpaneels (100-2, 100-3) verschweißt ist,
(ii) zwei laterale Seiten jedes Seitenpaneels (100-2, 100-3) jeweils mit verlängerten Längsseiten des genannten Hauptpaneels (100-1) verschweißt sind, um vertikale Eckverbindungen (100-4...100-7) zu bilden, die seitwärts um 0,05 Meter (2 Zoll) gefaltet und über Falten für zusätzliche Festigkeit verschweißt sind.

11. Nahtloses, verschweißtes, HDPE-Substrat-Tetra-LDPE-lagiges Wurmkompostierungsbett (100) nach Anspruch 1, wobei
(i) die genannten Fenster (70-1, 70-2, 70-3, 70-4, 70-5, 70-6) sich jeweils 0,11 Meter (4,5 Zoll) von der genannten jeweiligen benachbarten Tasche [(80-1, 80-2), (80-3, 80-4), (80-5, 80-6), (80-7, 80-8), (80-9, 80-10), (80-11, 80-12)] in der Form eines umgekehrten U entfernt befinden, und
(ii) die genannte Tasche (80-1, 80-6, 80-7, 80-12) in der Form eines umgekehrten U sich jeweils 0,12 Meter (5 Zoll) von der genannten jeweiligen vertikalen Eckverbindung (100-4, 100-5, 100-6, 100-7) entfernt befindet.

12. Nahtloses, verschweißtes, HDPE-Substrat-Tetra-LDPE-lagiges Wurmkompostierungsbett nach den Ansprüchen 1, 2 und 3, wobei
(a) jede genannte Tasche (80-1...80-12) in der Form eines umgekehrten U an einem U-förmigen einen Ende, zusammen mit dem genannten Seitenpaneel (100-2, 100-3), mit dem genannten Hauptpaneel (100-1) verschweißt und an dem genannten geschlossenen anderen Ende über die verseilte Oberseite der genannten Seitenpaneele (100-2, 100-3) durch Ausschneiden eines U-förmigen Abschnitts von dem geschlossenen anderen Ende und Verschweißen desselben mit dem genannten Seitenpaneel (100-2, 100-3) verankert ist;
(b) jede genannte Tasche (80-13, 80-14) in der Form eines umgekehrten U am geschlossenen anderen Ende über die verseilte Oberseite des genannten Hauptpaneels (100-1) durch Ausschneiden eines U-förmigen Abschnitts von dem geschlossenen anderen Ende und Verschweißen desselben mit dem genannten Hauptpaneel (100-1) verankert ist.

13. Nahtloses, verschweißtes, HDPE-Substrat-Tetra-LDPE-lagiges Wurmkompostierungsbett (100) nach Anspruch 1, installiert auf einem gut ausgelegten Boden (95) mit einem Gradienten von 0,15 Meter (6 Zoll) über 3,65 Meter (12 Fuß) durch
(i) korrektes Markieren von vierzehn Stellen zum Vergießen der genannten vierzehn Holzpfähle (81-1...81-14) in einem zur Installation gewählten Bereich, wobei der genannte gewählte Bereich des genannten gut ausgelegten Bodens (95) die Maße 4,57 Meter x 1,82 Meter (15 Fuß x 6 Fuß) hat;
(ii) Ausgraben der genannten markierten Positionen zu vierzehn Löchern (82-1...82-14), jedes genannte Loch mit den Maßen 0,07 Meter (3 Zoll) Durchmesser x 0,45 Meter (18 Zoll) Tiefe;
(iii) Vergießen jedes genannten Holzpfahls (81-1...81-14) in jedem genannten gegrabenen Loch (82-1...82-14), so dass jeder genannte Pfahl (81-1...81-14) über dem genannten gut ausgelegten Boden (95) bis zu einer Höhe von 0,53 Metern (21 Zoll) aufsteht;
(iv) gleichzeitiges Einführen der genannten Taschen (80-1...80-14) auf die genannten jeweiligen Holzpfähle (81-1...81-14) des genannten Tetra-Wurmbetts (100) und langsames Abgleitenlassen des genannten Tetra-Wurmbetts (100) über die genannten Holzpfähle (81-1...81-14), bis das genannte Tetra-Wurmbett (100) den gut ausgelegten Boden (95) über seine gesamte Peripherie berührt;
(v) Verlegen des genannten Abflussrohrs (83) unter dem genannten Abfluss (71), so dass das genannte Tetra-Wurmbett (100) auf dem genannten halbkreisförmigen, aufgeschnittenen Abschnitt (83-A) des genannten Abflussrohrs (83) sitzt, und Platzieren des anderen Endes (83-B) des genannten Abflussrohrs (83) über einem Container (84), der in einer ausgegrabenen Grube (85) platziert ist, um Wurmwaschflüssigkeit (86) aufzufangen.

14. Nahtloses, verschweißtes, HDPE-Substrat-Tetra-LDPE-lagiges Wurmkompostierungsbett (100) nach Anspruch 1, wobei vier Ösen (100-10 bis 100-13) an den oberen vier Eckpunkten des genannten Tetra-Wurmbetts (100) zum Verseilen der oberen Ränder der genannten Seitenpaneele (100-2, 100-3) und des genannten Hauptpaneels (100-1) vorgesehen sind, wobei die genannten Ösen (100-10...100-13) mit Faltklappen (100-14 bis 100-17) bedeckt sind, zum Schützen der Seile vor Schäden durch die Ränder der genannten Ösen (100-10 bis 100-13).

## Revendications

1. Une litière de lombricompostage, à couches de LDPE à structure tétra, à substrat de HDPE, thermoscellée, sans couture (100), constituée d'un tissu composite (90), ledit tissu composite (90) étant composé de quatre couches de LDPE, enduites par extrusion à chaud, stabilisées aux UV, lesdites quatre couches étant stratifiées et en alternance avec trois couches de substrat de HDPE (tissu tissé), stabilisées aux UV, ladite litière de lombricompostage à structure tétra (100) comprenant :
(i) un panneau principal (100-1) dudit tissu composite (90), mesurant 5,105 mètres x 0,60 mètre (16 pieds 9 pouces x 2 pieds) ;
(ii) deux panneaux latéraux (100-2, 100-3), chacun desdits panneaux latéraux dudit tissu composite (90), mesurant 3,88 mètres x 0,60 mètre (12 pieds 9 pouces x 2 pieds) de telle sorte que
(iii)
(a) les dimensions finales de ladite litière de lombricompostage à structure tétra (100), composée dudit tissu composite à sept couches (90) font 12 pieds de long x 4 pieds de large x 2 pieds de haut ;
(b) ladite litière de lombricompostage (100) a une capacité volumétrique interne pour contenir un volume de 2,714 mètres cubes (96 pieds cubes) de déchets ; et
(c) la couleur de ladite litière de lombricompostage à structure tétra (100) est verte à l'extérieur et blanc neige à l'intérieur ;
(iv) quatorze poches en forme de U inversé (80-1 .... 80-14) composées dudit tissu composite à sept couches (90), chacune desdites poches étant ouverte par une embouchure en forme de U inversé à l'extrémité inférieure de ladite poche et fermée à l'extrémité supérieure de ladite poche par l'ancrage de l'extrémité avec corde desdits panneaux latéraux (100-2, 100-3) ou dudit panneau principal (100-1) ;
(v) quatorze poteaux en bois, ronds, droits, de 0,99 mètre (39 pouces) de long (81-1.... 81-14), chacun desdits poteaux étant cimenté dans des trous pré-marqués (82-1 .... 82-14) à 0,45 mètre (18 pouces) de profondeur sous la surface du sol préparé (95) et dépassant au-dessus de la surface du sol (95) sur une hauteur de 0,5 mètre (21 pouces) ;
(vi) six fenêtres (70-1 .... 70-6) créées en découpant la partie pré-marquée respective desdits panneaux latéraux (100-2, 100-3) et en thermoscellant un filet en HDPE sur lesdites parties découpées avec des angles arrondis, chacune desdites fenêtres mesurant 0,20 mètre x 0,11 mètre (8 pouces x 4,5 pouces) ;
(vii) une évacuation (71), découpée dans une partie marquée dudit panneau principal (100-1) et recouverte d'un filet en HDPE sur l'ouverture de l'évacuation (71), ladite évacuation (71) reposant au-dessus d'un tuyau découpé semi-circulaire (83) le long d'une partie de la longueur (83-A) et enterré sur le reste de la longueur (83-B) dans le sol sous ladite évacuation (71) de ladite litière de lombricompostage à structure tétra (100), ladite évacuation (71) mesurant 0,088 mètre x 0,03 mètre (3,5 pouces x 1,5 pouce).

2. Une litière de lombricompostage, à couches de LDPE à structure tétra, à substrat de HDPE, thermoscellée, sans couture (100) selon la revendication 1, dans laquelle lesdites six poches (80-1 .... 80-6)
(a) mesurent 0,60 mètre x 0,20 mètre (24 pouces x 8 pouces) ; et
(b) sont thermoscellées sur ledit côté extérieur vert dudit panneau latéral (100-2) à une position perpendiculaire à l'axe longitudinal dudit panneau latéral (100-2) de ladite litière de lombricompostage à structure tétra (100).

3. Une litière de lombricompostage, à couches de LDPE à structure tétra, à substrat de HDPE, thermoscellée, sans couture (100) selon la revendication 1, dans laquelle lesdites six autres poches (80-7 .... 80-12)
(a) mesurent 0,60 mètre x 0,20 mètre (24 pouces x 8 pouces) ; et
(b) sont thermoscellées sur ledit côté extérieur vert dudit panneau latéral (100-3) à une position perpendiculaire à l'axe longitudinal dudit panneau latéral (100-3) de ladite litière de lombricompostage à structure tétra (100).

4. Une litière de lombricompostage, à couches de LDPE à structure tétra, à substrat de HDPE, thermoscellée, sans couture (100) selon la revendication 1, dans laquelle ladite treizième poche (80-13)
(a) mesure 0,60 mètre x 0,20 mètre (24 pouces x 8 pouces) ; et
(b) est thermoscellée sur ladite surface extérieure verte et est près d'une partie latérale dudit panneau principal (100-1) à une position parallèle à l'axe longitudinal dudit panneau principal (100-1).

5. Une litière de lombricompostage, à couches de LDPE à structure tétra, à substrat de HDPE, thermoscellée, sans couture (100) selon la revendication 1, dans laquelle ladite quatorzième poche (80-14)
(a) mesure 0,60 mètre x 0,20 mètre (24 pouces x 8 pouces) ; et
(b) est thermoscellée sur ladite surface extérieure verte et près de ladite autre partie latérale dudit panneau principal (100-1) à une position parallèle à l'axe longitudinal dudit panneau principal (100-1).

6. Une litière de lombricompostage, à couches de LDPE à structure tétra, à substrat de HDPE, thermoscellée, sans couture (100) selon la revendication 1, dans laquelle lesdites fenêtres (70-1 .... 70-3) sont découpées au centre dudit panneau latéral (100-2) en des ouvertures de 0,20 mètre x 0,11 mètre (8 pouces x 4,5 pouces) avec des angles arrondis qui sont thermoscellés avec des filets en HDPE.

7. Une litière de lombricompostage, à couches de LDPE à structure tétra, à substrat de HDPE, thermoscellée, sans couture (100) selon la revendication 1, dans laquelle lesdites fenêtres (70- 4 .... 70-6) sont découpées au centre dudit panneau latéral (100-3) en des ouvertures de 0,20 mètre x 0,11 mètre (8 pouces x 4,5 pouces) avec des angles arrondis qui sont thermoscellés avec des filets en HDPE.

8. Une litière de lombricompostage, à couches de LDPE à structure tétra, à substrat de HDPE, thermoscellée, sans couture (100) selon les revendications 1 et 2 à 5, dans laquelle lesdites poches (80-1 .... 80-14) sont thermoscellées sur deux côtés (80- A) desdites poches (80-1 .... 80-14) sur un dégagement de 0,15 mètre (6 pouces) (80-B) le long de la longueur totale desdites poches (80-1 .... 80-14).

9. Une litière de lombricompostage, à couches de LDPE à structure tétra, à substrat de HDPE, thermoscellée, sans couture (100) selon la revendication 1, dans laquelle
(a) lesdits panneaux latéraux (100-2, 100-3) sont pliés par une corde sur 0,05 mètre (2 pouces) à une extrémité longitudinale qui est la plus éloignée de ladite embouchure en forme de U inversé desdites poches (80-1 .... 80-12) ; et
(b) ledit panneau principal (100-1) est plié par une corde sur 0,05 mètre (2 pouces) aux deux extrémités latérales qui sont les plus éloignées de ladite embouchure en forme de U inversé desdites poches (80-13, 80-14).

10. Une litière de lombricompostage, à couches de LDPE à structure tétra, à substrat de HDPE, thermoscellée, sans couture (100) selon la revendication 1, dans laquelle
(i) chacun desdits panneaux latéraux (100-2, 100-3) à un côté longitudinal sans corde est thermoscellé avec un côté longitudinal dudit panneau principal (100-1)
(ii) deux partie latérales de chacun des panneaux latéraux (100-2, 100-3) sont chacune thermoscellée avec des côtés longitudinaux étendus dudit panneau principal (100-1) pour former des jonctions angulaires verticales (100-4 .... 100-7) qui sont pliées latéralement sur 0,05 mètre (2 pouces) et thermoscellées sur les plis pour une résistance supplémentaire.

11. Une litière de lombricompostage, à couches de LDPE à structure tétra, à substrat de HDPE, thermoscellée, sans couture (100) selon la revendication 1, dans laquelle
(i) chacune desdites fenêtres (70-1, 70-2, 70-3, 70-4, 70-5, 70-6) est à 0,11 mètre (4,5 pouces) de ladite poche en forme de U inversé adjacente [(80-1, 80-2), (80-3, 80-4), (80-5, 80-6), (80-7, 80-8), (80-9, 80-10), (80-11, 80-12)] respectivement, et
(ii) chacune desdites poches en forme de U inversé (80-1, 80-6, 80-7, 80-12) est à 0,12 mètre (5 pouces) de ladite jonction angulaire verticale (100-4, 100-5, 100-6, 100-7) respectivement.

12. Une litière de lombricompostage, à couches de LDPE à structure tétra, à substrat de HDPE, thermoscellée, sans couture selon les revendications 1, 2 et 3, dans laquelle
(a) chacune desdites poches en forme de U inversé (80-1 .... 80-12) à une extrémité en forme de U est thermoscellée - ainsi que ledit panneau latéral (100-2, 100-3) - audit panneau principal (100-1) et, à ladite autre extrémité fermée est ancrée sur la partie supérieure à corde desdits panneaux latéraux (100-2, 100-3) en découpant une partie en forme de U de l'autre extrémité fermée et en la thermoscellant avec ledit panneau latéral (100-2, 100-3) ;
(b) chacune desdites poches en forme de U inversé (80-13, 80-14) à l'autre extrémité fermée est ancrée sur la partie supérieure à corde dudit panneau principal (100-1) en découpant une partie en forme de U de l'autre extrémité fermée et en la thermoscellant avec ledit panneau principal (100-1).

13. Une litière de lombricompostage, à couches de LDPE à structure tétra, à substrat de HDPE, thermoscellée, sans couture (100), selon la revendication 1, est installée sur un sol bien préparé (95) ayant une inclinaison de 0,15 mètre (6 pouces) sur une distance de 3,65 mètres (12 pieds), l'installation consistant à
(i) marquer correctement quatorze emplacements pour cimenter lesdits quatorze poteaux en bois (81-1 ... 81-14) sur une zone sélectionnée pour l'installation, ladite zone sélectionnée dudit sol bien préparé (95) mesurant 4,57 mètres x 1,82 mètre (15 pieds x 6 pieds) ;
(ii) creuser quatorze trous (82-1 .... 82-14) auxdites positions marquées, chaque trou mesurant 0,07 mètre (3 pouces) de diamètre x 0,45 mètre (18 pouces) de profondeur ;
(iii) cimenter chacun desdits poteaux en bois (81-1 .... 81-14) dans chacun desdits trous creusés (82-1 .... 82-14) de telle sorte que chacun desdits poteaux (81-1 .... 81-14) dépasse au-dessus du sol bien préparé (95) d'une hauteur de 0,53 mètre (21 pouces) ;
(iv) introduire lesdites poches (80- 1 .... 80-14) sur lesdits poteaux en bois respectifs (81-1 .... 81-14) de ladite litière de lombricompostage à structure tétra (100) simultanément et faire coulisser graduellement ladite litière de lombricompostage à structure tétra (100) sur lesdits poteaux en bois (81-1 .... 81-14) jusqu'à ce que ladite litière de lombricompostage à structure tétra (100) touche ledit sol bien préparé (95) tout le long de sa périphérie ;
(v) monter sous ladite évacuation (71) ledit tuyau d'évacuation (83) de telle sorte que ladite litière de lombricompostage à structure tétra (100) repose au-dessus de ladite partie découpée semi-circulaire (83-A) dudit tuyau d'évacuation (83) et placer l'autre extrémité (83-B) dudit tuyau d'évacuation (83) au-dessus d'un contenant (84), placé dans une fosse creusée (85) pour collecter le liquide de lavage du lombricompostage (86).

14. Une litière de lombricompostage, à couches de LDPE à structure tétra, à substrat de HDPE, thermoscellée, sans couture (100), selon la revendication 1, dans laquelle quatre oeillets (100-10 à 100-13) sont fournis aux quatre points d'angle supérieurs de ladite litière de lombricompostage à structure tétra (100) pour attacher par cordage les bords supérieurs desdits panneaux latéraux (100-2, 100-3) et dudit panneau principal (100-1), lesdits oeillets (100-10 .... 100-13) étant recouverts de rabats repliables (100-14 à 100-17) pour protéger le cordage contre tout endommagement par les bords desdits oeillets (100-10 à 100-13).
